(19) Europäisches Patentamt
European Patent Office
Office européen des brevets

(11) Numéro de publication: **0 148 057**
**B1**

(12) **FASCICULE DE BREVET EUROPEEN**

(45) Date de publication du fascicule du brevet: **18.10.89**

(51) Int. Cl.⁴: **C 08 B 37/08**

(21) Numéro de dépôt: **84402495.0**

(22) Date de dépôt: **05.12.84**

(54) **Chitosane 6-sulfate et procédé pour sa préparation.**

(30) Priorité: **06.12.83 FR 8319506**

(43) Date de publication de la demande: **10.07.85 Bulletin 85/28**

(45) Mention de la délivrance du brevet: **18.10.89 Bulletin 89/42**

(84) Etats contractants désignés: **AT BE CH DE FR GB IT LI LU NL SE**

(56) Documents cités:
**EP-A- 0 116 251**
**FR-A- 1 093 999**
**US-A- 2 755 275**

**CHEMICAL ABSTRACTS, vol. 87, no. 1, 4 juillet 1977, page 35, réf. no. 381w, Columbus, Ohio, US; & JP - A - 76 06 720 (SEIKAGAKU KOGYO CO., LTD.) 01.03.1976**

(73) Titulaire: **SANOFI, 40, Avenue George V, F-75008 Paris (FR)**

(72) Inventeur: **Naggi, Annamaria, Via Monte Nevoso N.32, Legnano Milano (IT)**
Inventeur: **Torri, Giangiacomo, Via Confalonieri 8, Bergamo (IT)**

(74) Mandataire: **Gillard, Marie-Louise, Cabinet Beau de Loménie 55, Rue d'Amsterdam, F-75008 Paris (FR)**

**EP 0 148 057 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne un chitosane 6-sulfate et un procédé pour sa préparation.

Le chitosane est un polysaccharide constitué par 400 à 6000 unités de D-bêta-(1,4)-glucosamine contenant de zéro à un maximum de 30% de radicaux acétyle fixés sur le groupe amino de la glucosamine.

Des méthodes pour la sulfatation du chitosane sont décrites en littérature. Ces méthodes donnent des sulfates de chitosane ayant des groupes sulfate fixés au hasard sur les groupes hydroxy en positions 3 et 6 et sur le groupe amino en position 2 de l'unité de glucosamine.

Le brevet français 1 093 099 décrit un procédé pour la préparation du sulfate de chitosane par réaction du chitosane avec un agent de sulfatation. Selon ledit brevet, l'agent de sulfatation peut être un mélange d'anhydride sulfurique et d'anhydride sulfureux ou d'autres agents de sulfatation comme l'acide chlorosulfonique dans la pyridine ou l'acide sulfurique fumant.

Les exemples du brevet français ci-dessus décrivent la réaction avec un mélange d'anhydride sulfurique et d'anhydride sulfureux donnant un sulfate de chitosane ayant un pourcentage en soufre de 14,8%, 9%, 16,5%, 14,7% et 17,56% (en considérant un produit complètement désacétylé) correspondant à un degré de substitution (groupes sulfate par unité de glucosamine) de 1,22, 0,75, 1,36, 1,21 et, respectivement 1,45.

Le brevet ne précise pas où le groupe sulfate est introduit, mais il a été démontré postérieurement que la réaction avec le mélange anhydride sulfurique/anhydride sulfureux introduit le groupe sulfate également sur le groupe 2-amino du chitosane.

Un exemple du même brevet décrit spécifiquement la réaction du chitosane avec l'acide chlorosulfonique dans la pyridine. Une telle réaction donne cependant un produit ayant le groupe sulfate sur l'amine en position 2. Par conséquent, le procédé n'est pas sélectif pour la position 6.

Le brevet US 2 755 275 revendique un procédé pour la sulfatation de la chitine par réaction du produit de départ avec l'acide chlorosulfonique ou l'anhydride sulfurique dans un solvant halogéné. Dans la description dudit brevet, il est indiqué que le chitosane peut être également utilisé comme produit de départ. Toutefois, le procédé revendiqué provoque, dans le cas du chitosane, une sulfatation parallèle du groupe amino en position 2.

Nagasawa et al. (Chem. Pharm. Bull. 1972, 20, 157–162 – C.A. 76, 113456r) décrivent la sulfatation du chitosane par action de l'acide sulfurique concentré. Les auteurs indiquent clairement que le composé ainsi obtenu est presque complètement N-sulfaté.

En général, la sulfatation du chitosane selon les méthodes connues donne donc des produits partiellement ou complétement sulfatés sur le groupe amino en position 2 et, en même temps, sur les groupes hydroxy en positions 3 et 6.

Aucun des procédés décrits dans la littérature ne permet la préparation d'un chitosane ayant le groupe hydroxy en position 6 de pratiquement toutes les unités de glucosamine sélectivement sulfaté et, en même temps, n'ayant aucun groupe sulfate dans les positions 2 et 3.

Ainsi, aucun chitosane 6-sulfate n'ayant aucun groupe sulfate sur les fonctions 2-amino et 3-hydroxy des unités de glucosamine n'a été décrit précédemment.

Il a maintenant été trouvé que, par traitement d'un chitosane avec un mélange d'acide sulfurique et d'acide chlorosulfonique, on obtient une sulfatation sélective du groupe hydroxyle en position 6.

On a aussi trouvé qu'en faisant réagir un chitosane avec un mélange acide sulfurique/acide chlorosulfonique on peut obtenir en même temps une dépolymérisation et une sulfatation.

Il a été également trouvé que la dépolymérisation ne comporte aucune sulfatation ultérieure et que l'on obtient avec de très bons rendements un chitosane dépolymérisé qui est sélectivement sulfaté en position 6.

Ainsi la présente invention concerne, selon un de ses aspects, un chitosane 6-sulfate de formule:

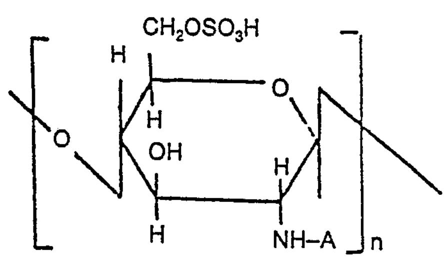

dans laquelle n est un nombre entier de 4 à 6000 et A représente un atome d'hydrogène ou, dans un nombre allant jusqu'à 30% des n unités, un groupe acétyle, ainsi que ses sels avec des acides inorganiques ou organiques, avec des bases inorganiques ou organiques et ses sels internes.

L'expression «n est un numéro entier de 4 à 6000» signifie que dans la structure I ci-dessus l'unité saccharidique peut être répétée 4 à 6000 fois selon une courbe du type gaussien.

Le chitosane de départ étant préparé par déacétylation de la chitine, la masse moléculaire apparente, à savoir la valeur de n, et le pourcentage des groupes acétyle dépendent du procédé utilisé pour la préparation dudit chitosane de départ.

Contrairement aux sulfates de chitosane connus, dans lesquels les groupes sulfate sont distribués au hasard aussi bien dans les unités de glucosamine que dans les n unités du polysaccharide, le chitosane 6-sulfate de la présente invention a une structure bien définie qui montre un seul groupe sulfate par unité de glucosamine.

En outre, contrairement aux sulfates de chitosane connus dans lesquels il y a une disproportion entre les groupes sulfate et les groupes amino et dans lesquels, en plus, les groupes amino sont en général sulfatés, les chitosanes 6-sulfate de la

présente invention montrent un rapport groupes sulfate : groupe amino variable de 1 : 1 à 1 : 0,7 selon le nombre des groupes acétyle des chitosanes de départ.

Les composés de la présente invention sont des ampholytes ayant des propriétés polyélectrolytiques et sont donc utiles comme séquestrants des ions métalliques, comme agents de dispersion et comme agents épaississants pour l'industrie textile, cosmétique et alimentaire, ainsi que comme ingrédients actifs dans des compositions pharmaceutiques à action biologique tamponnante et, plus particulièrement, avec une activité antipeptique, antiacide et protectrice de la muqueuse gastrique.

Selon un autre de ses aspects, la présente invention a pour objet un procédé pour la préparation d'un chitosane 6-sulfate, plus particulièrement représenté par la formule I ci-dessus, caractérisé en ce qu'un chitosane est traité avec un mélange d'acide sulfurique et d'acide chlorosulfonique et le produit ainsi obtenu est isolé sous forme de sel alcalin ou transformé en la forme acide ou en un autre sel.

Les deux acides, dans le mélange, sont concentrés et de préférence leur concentration est d'au moins 95% en poids.

Le rapport acide sulfurique : acide chlorosulfonique varie de 4 : 1 à 1 : 4, un rapport d'environ 2 : 1 étant particulièrement préféré.

La température de réaction peut varier entre −20 °C et −40 °C; de préférence, le produit de départ est introduit dans le mélange d'acides à basse température. Après une période variable de 5 min à 2 heures, selon la température de réaction, la réaction est achevée et le chitosane 6-sulfate est isolé selon les méthodes classiques. De préférence, le mélange réactionnel est versé dans un solvant apolaire et aprotique, tel que l'éther diétylique, où le chitosane 6-sulfate est insoluble, et le produit obtenu est neutralisé et purifié par dialyse.

Le poids moléculaire du produit final dépend du temps de réaction et de la teneur en eau du mélange de réaction.

Le chitosane 6-sulfate ainsi obtenu est isolé sous forme de sel alcalin selon les techniques classiques, par exemple par lyophilisation ou évaporation sous pression réduite et caractérisé par les méthodes physico-chimiques connues.

D'autres sels, par exemple, le sel de calcium, peuvent être obtenus à partir des sels alcalins, le sel de sodium de préférence, par réaction d'échange avec un sel approprié, par exemple un sel de calcium, en utilisant éventuellement une résine échangeuse d'ions.

Le poids moléculaire des chitosanes de départ préférés peut varier (selon la littérature, pour des préparations commerciales) de 100 000 jusqu'à 800 000 équivalents à environ 400 à 3400 unités saccharidiques. Aucun groupe terminal de ces produits ne peut être détecté par spectroscopie RMN.

Comme produit de départ, on peut aussi utiliser un chitosane préalablement dépolymérisé par des méthodes connues. L'action du mélange acide sulfurique/acide chlorosulfonique peut comporter, à côté de la sulfatation quantitative et sélective en position 6, une dépolymérisation ultérieure.

Les exemples suivants illustrent l'invention sans toutefois la limiter.

Exemple 1

A un mélange de 20 ml d'acide sulfurique à 95% et 10 ml d'acide chlorosulfonique, préalablement refroidi à une température d'environ 0–4 °C, on ajoute 500 mg de chitosane ANIC, lot 116, contenant 15 à 20% de groupes N-acétyle, et on agite le mélange à la même température pendant environ 1 h. On verse ensuite le mélange réactionnel dans de l'éther diéthylique préalablement refroidi, on filtre le précipité ainsi obtenu et on le neutralise avec une solution de bicarbonate de potassium. Après une dialyse dans des tubes à 8500 D, on obtient un chitosane 6-sulfate (P 211–SR 95338) ayant les caractéristiques suivantes:

– P.M.: environ 10 000

– Degré de substitution (par voie conductimétrique): 1.

– Spectre IR: Large bande dans la région 1300–1200 $cm^{-1}$, caractéristique des groupes sulfates.

– Spectre 13C-RMN: la figure 1 montre la complète disparition du signal relatif au groupe hydroxyle primaire et l'apparition du signal relatif aux groupes 6-O-sulfate. Aucun signal de N-sulfate ou de 3-O-sulfate.

Exemple 2

a) A une solution de 1 g de chitosane ANIC, lot 116, dans 50 ml d'acide acétique à 30%, on ajoute 2,3 ml d'acide nitreux 0,5 M préparé à partir de 10 ml de nitrite de baryum monohydraté 0,5 M et 10 ml d'acide sulfurique 0,5 M, puis on agite à la température ambiante pendant 12 h, on concentre sous pression réduite et l'on précipite avec de l'acétone. On filtre le précipité, on le lave avec de l'acétone, on le sèche, on le dissout dans de l'eau et on le traite avec 30 mg de borohydrure de sodium. Après 12 h à la température ambiante, on détruit l'excès de borohydrure de sodium avec de l'Amberlite IR 1200 H+ et on élimine l'acide borique par évaporation sous pression réduite en présence de méthanol. On obtient ainsi un chitosane dépolymérisé ayant un poids moléculaire (environ 2500) très inférieur à celui du chitosane de départ.

b) A un mélange de 20 ml d'acide sulfurique à 95% et de 10 ml d'acide chlorosulfonique à 98%, refroidi à −4–0 °C, on ajoute 1 g de chitosane dépolymérisé obtenu comme décrit ci-dessus. On laisse le mélange réactionnel à la même température pendant 1 h, on le verse dans 250 ml d'éther diéthylique préalablement refroidi, on filtre le précipité et on le lave avec de l'éther diéthylique froid. On dissout le produit dans de l'eau et l'on neutralise avec une solution d'hydroxyde de sodium 0,5 M. Après dessalification par chromatographie sur Sephadex G25, on obtient, avec un rendement de 90%, un chitosane 6-sulfate dépolymérisé ayant les caractéristiques suivantes:

– P.M.: 2000

– Degré de substitution: 1.

– Spectre IR: Large bande dans la région 1300–1200 $cm^{-1}$, caractéristique des groupes sulfates.

– Spectre 13C-RMN: disparition du signal relatif au groupe hydroxyle primaire et apparition d'un nouveau signal dû au groupe sulfate.

**Revendications pour les Etats contractants: BE, CH, DE, FR, GB, IT, LI, LU, NL, SE**

1. Chitosane 6-sulfate de formule:

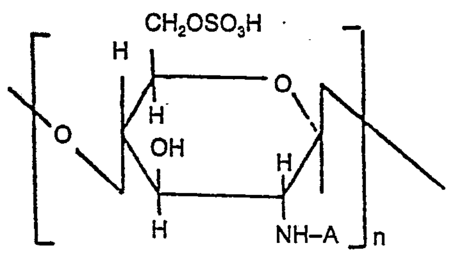

dans laquelle n est un nombre entier de 4 à 6000 et A représente un atome d'hydrogène ou, dans un nombre allant jusqu'à 30% des n unités, un groupe acétyle ou son sel avec un acide inorganique ou organique ou avec une base inorganique ou organique ou son sel interne.

2. Procédé pour la préparation d'un chitosane 6-sulfate selon la revendication 1, caractérisé en ce qu'un chitosane est ajouté, à la température de −20 à +40 °C, à un mélange d'acide sulfurique et d'acide chlorosulfonique dans un rapport de 4 : 1 à 1 : 4 et, en ce qu'après une période de 5 minutes à 2 heures, le produit est isolé sous forme de sel alcalin ou transformé en la forme acide ou en autre sel.

3. Procédé selon la revendication 2, caractérisé en ce que la concentration des deux acides est d'au moins 95% en poids.

4. Procédé selon l'une des revendications 2 ou 3, caractérisé en ce que le rapport acide sulfurique : acide chlorosulfonique est d'environ 2 : 1.

5. Procédé selon l'une quelconque des revendications 2 à 4, caractérisé en ce que le chitosane de départ est préalablement dépolymérisé.

**Revendications pour l'Etat contractant: AT**

1. Procédé pour la préparation d'un chitosane 6-sulfate de formule:

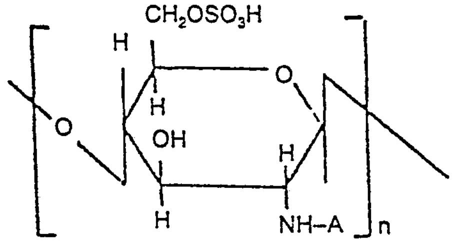

dans laquelle n est un numéro entier de 4 à 6000 et A représente un atome d'hydrogène ou, dans un nombre allant jusqu'à 30% des n unités, un groupe acétyle, de sels avec des acides inorganiques ou organiques ou avec des bases inorganiques ou organiques ou de ses sels internes, caractérisé en ce qu'un chitosane est ajouté, à la température de −20 à +40 °C, à un mélange d'acide sulfurique et d'acide chlorosulfonique dans un rapport de 4 : 1 à 1 : 4 et en ce qu'après une période de 5 minutes à 2 heures, le produit est isolé sous forme de sel alcalin ou transformé en la forme acide ou en autre sel.

2. Procédé selon la revendication 1, caractérisé en ce que la concentration des deux acides est d'au moins 95% en poids.

3. Procédé selon l'une des revendications 1 ou 2, caractérisé en ce que le rapport acide sulfurique : acide chlorosulfonique est d'environ 2 : 1.

4. Procédé selon l'une quelconque revendications 1 à 3, caractérisé en ce que le chitosane de départ est préalablement dépolymérisé.

**Claims for contracting States: BE, CH, DE, FR, GB, IT, LI, LU, NL, SE**

1. Chitosan 6-sulfate of formula

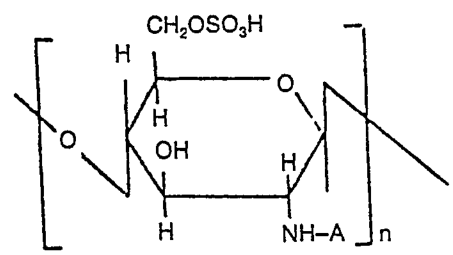

wherein n is an integer from 4 to 6000 and A represents a hydrogen atom or, in a number of up to 30% of the n subunits, an acetyl group or a salt thereof with an inorganic or organic acid or with an inorganic or organic bases or an internal salt thereof.

2. Process for the preparation of a chitosan 6-sulfate as claimed in claim 1, characterized in that a chitosan is added, at the temperature of −20 to +40 °C, to a mixture of sulfuric acid and chlorosulfonic acid in a ratio of 4 : 1 to 1 : 4 and in that, after a period of from five minutes to two hours, the product is isolated as an alkali metal salt or converted into the acid from or in another salt.

3. Process according to claim 2, characterized in that the concentration of the two acids is at least 95% by weight.

4. Process according to one of claims 2 or 3, characterized in that the ratio sulfuric acid : chlorosulfonic acid is about 2 : 1.

5. Process according to any one of claims 2 to 4, characterized in that the starting chitosan is previously depolymerized.

**Claims for the contracting State: AT**

1. Process for the preparation of a chitosan 6-sulfate of formula

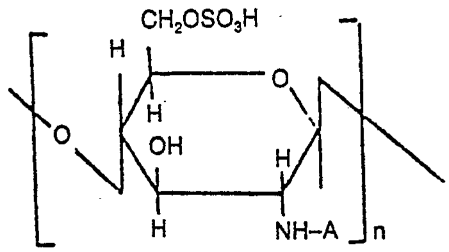

wherein n is an integer from 4 to 6000 and A represents a hydrogen atom or, in a number of up to 30% of the n subunits, an acetyl group or a salt thereof with an inorganic or organic acid or with an inorganic or organic bases or internal salts thereof, characterized in that a chitosan is added, at the temperature of −20 to +40 °C to a mixture of sulphuric acid and chlorosulfonic acid in a ratio of 4 : 1 to 1 : 4 and in that after a period of 5 mins. to 2 hours, the product is isolated as an alkali metal salt or converted into the acid form or in another salt.

2. Process according to claim 1, characterized in that the concentration of the two acids is at least 95% by weight.

3. Process according to one of claims 1 or 2, characterized in that the ratio sulfuric acid : chlorosulfonic acid is about 2 : 1.

4. Process according to any one of claims 1 to 3, characterized in that the starting chitosan is previously depolymerized.

**Patentansprüche für die Vertragsstaaten: BE, CH, DE, FR, GB, IT, LI, LU, NL, SE**

1. 6-Chitosansulfat der Formel

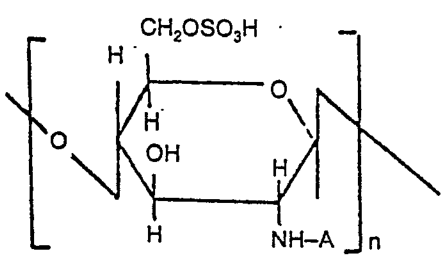

worin n eine ganze Zahl von 4 bis 6000 ist und A für ein Wasserstoffatom steht oder bei einer Zahl bis zu 30% der n-Einheiten eine Acetylgruppe darstellt, oder das Salz davon mit einer anorganischen oder organischen Säure oder mit einer anorganischen oder organischen Base oder das innere Salz.

2. Verfahren zur Herstellung eines 6-Chitosansulfats nach Anspruch 1, dadurch gekennzeichnet, daß ein Chitosan bei einer Temperatur von −20 bis +40 °C zu einer Mischung aus Schwefelsäure und Chlorsulfonsäure in einem Verhältnis von 4 : 1 bis 1 : 4 zugegeben wird und daß das Produkt nach einem Zeitraum von 5 Minuten bis 2 Stunden in Form des Alkalisalzes isoliert oder in saure Form oder in ein anderes Salz übergeführt wird.

3. Verfahren nach Anspruch 2, dadurch gekennzeichnet, daß die Konzentration der beiden Säuren mindestens 95 Gew.% beträgt.

4. Verfahren nach einem der Ansprüche 2 oder 3, dadurch gekennzeichnet, daß das Verhältnis Schwefelsäure : Chlorsulfonsäure etwa 2 : 1 beträgt.

5. Verfahren nach einem der Ansprüche 2 bis 4, dadurch gekennzeichnet, daß das Ausgangschitosan zuvor depolymerisiert wird.

**Patentansprüche für den Vertragsstaat: AT**

1. Verfahren zur Herstellung eines 6-Chitosansulfats der Formel

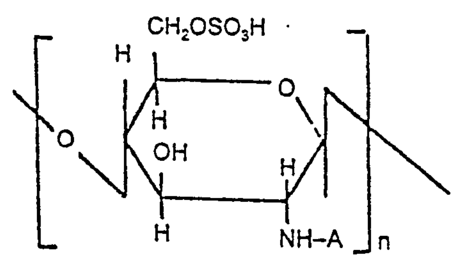

worin n eine ganze Zahl von 4 bis 6000 ist und A für ein Wasserstoffatom steht oder bei einer Zahl bis zu 30% der n-Einheiten eine Acetylgruppe darstellt, oder seiner Salze mit anorganischen oder organischen Säuren oder mit anorganischen oder organischen Basen oder seiner inneren Salze, dadurch gekennzeichnet, daß ein Chitosan bei einer Temperatur von −20 bis +40 °C zu einer Mischung aus Schwefelsäure und Chlorsulfonsäure in einem Verhältnis von 4 : 1 bis 1 : 4 zugegeben wird und daß das Produkt nach einem Zeitraum von 5 Minuten bis 2 Stunden in Form des Alkalisalzes isoliert oder in saure Form oder in ein anderes Salz übergeführt wird.

2. Verfahren nach Anspruch 1, dadurch gekennzeichnet, daß die Konzentration der beiden Säuren mindestens 95 Gew.% beträgt.

3. Verfahren nach einem der Ansprüche 1 oder 2, dadurch gekennzeichnet, daß das Verhältnis Schwefelsäure : Chlorsulfonsäure etwa 2 : 1 beträgt.

4. Verfahren nach einem der Ansprüche 1 bis 3, dadurch gekennzeichnet, daß das Ausgangschitosan zuvor depolymerisiert wird.

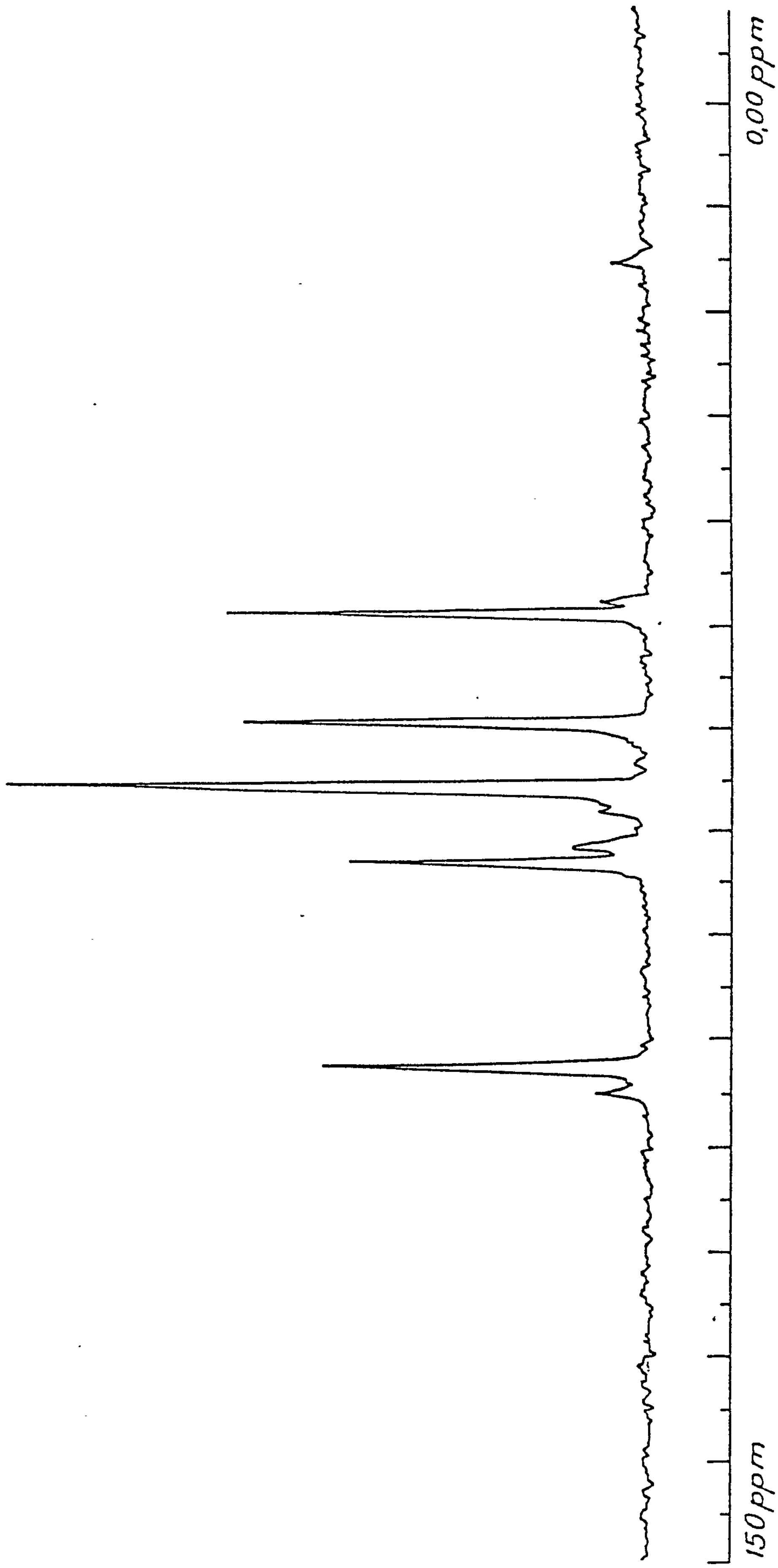
0,00 ppm
150 ppm